# EUROPEAN PATENT SPECIFICATION

(11) **EP 2 727 911 B1**
(45) Date of publication and mention of the grant of the patent: **21.12.2016**
(21) Application number: 12190851.1
(22) Date of filing: 31.10.2012
(51) Int. Cl.: C07D 217/06, A61K 31/472, A61P 33/06

(54) **Novel means and methods for treating malaria and other parasitic disorders**
Neuartiges Mittel und neuartige Verfahren zur Behandlung von Malaria und anderen parasitären Krankheiten
Nouveaux moyens et procédés de traitement du paludisme et autres troubles parasitaires

(43) Date of publication of application: 07.05.2014
(73) Proprietor: Medizinische Hochschule Hannover, 30625 Hannover (DE)
(72) Inventor: Manstein, Dietmar, 30559 Hannover (DE); Preller, Matthias, 30163 Hannover (DE); Kalesse, Markus, 31303 Burgdorf (DE)
(74) Representative: Vossius & Partner Patentanwälte Rechtsanwälte mbB

(56) References cited:
- WO-A1-00/31067
- WO-A1-01/60415
- WO-A1-2007/120083
- WO-A1-2010/024110
- WO-A2-2007/109783
- CN-A- 102 399 145
- DE-A1- 3 016 827
- US-A- 5 135 926
- US-A- 5 356 894
- JUNEK, RICHARD ET AL: "Antileukotrienic N-arylethyl-2-arylacetamides in the treatment of ulcerative colitis", EUROPEAN JOURNAL OF MEDICINAL CHEMISTRY , 42(8), 1084-1094 CODEN: EJMCA5; ISSN: 0223-5234, 2007, XP22165142,
- SCHIERHOLT, ALEXANDER ET AL: "Cysteine-Based Mannoside Glycoclusters: synthetic Routes and Antiadhesive Properties", EUROPEAN JOURNAL OF ORGANIC CHEMISTRY , (16), 3120-3128, S3120/1-S3120/14 CODEN: EJOCFK; ISSN: 1434-193X, 2010, XP009166307,
- WOODARD, SCOTT S. ET AL: "Combinatorial synthesis of 3,5-dimethylene substituted 1,2,4-triazoles", COMBINATORIAL CHEMISTRY & HIGH THROUGHPUT SCREENING , 14(2), 132-137 CODEN: CCHSFU; ISSN: 1386-2073, 2011, XP009166313,
- VISWANATHAN, C. L. ET AL: "Synthesis and evaluation of uterine relaxant activity for a novel series of substituted p-hydroxyphenylethanolamines", BIOORGANIC & MEDICINAL CHEMISTRY , 14(19), 6581-6585 CODEN: BMECEP; ISSN: 0968-0896, 2006, XP0027992937,
- KOLHATKAR, VIDULA ET AL: "Identification of novel nonsteroidal compounds as substrates or inhibitors of hASBT", JOURNAL OF PHARMACEUTICAL SCIENCES , 101(1), 116-126 CODEN: JPMSAE; ISSN: 0022-3549, 2012, XP009166306,
- GRIMBERG, BRIAN T. ET AL: "Addressing the malaria drug resistance challenge using flow cytometry to discover new antimalarials", BIOORGANIC & MEDICINAL CHEMISTRY LETTERS , 19(18), 5452-5457 CODEN: BMCLE8; ISSN: 0960-894X, 2009, XP026501183,
- CHAUDHARY, PREETI M. ET AL: "Exploration of click reaction for the synthesis of modified nucleosides as chitin synthase inhibitors", BIOORGANIC & MEDICINAL CHEMISTRY , 17(6), 2433-2440 CODEN: BMECEP; ISSN: 0968-0896, 2009, XP025981941,
- PARK, E.-S. ET AL: "Antimicrobial activity of phenol and benzoic acid derivatives", INTERNATIONAL BIODETERIORATION & BIODEGRADATION , 47(4), 209-214 CODEN: IBBIES; ISSN: 0964-8305, 2001, XP055038115,
- KUWAJIMA, HIROSHI ET AL: "Synthesis of 13C-labeled possible intermediates in the biosynthesis of phenylethanoid derivatives, cornoside and rengyosides", CHEMICAL & PHARMACEUTICAL BULLETIN , 46(4), 581-586 CODEN: CPBTAL; ISSN: 0009-2363, 1998, XP009166287,
- YAEGASHI TAKASHI ET AL: "Synthesis and structure-activity study of protease inhibitors. III. Amidinophenols and their benzoyl esters", CHEMICAL AND PHARMACEUTICAL BULLETIN,, vol. 32, no. 11, 1 January 1984 (1984-01-01), pages 4466-4477, XP002432864, ISSN: 0009-2363
- ODENBAUGH AMY L ET AL: "An investigation of antibody acyl hydrolysis catalysis using a large set of related haptens", BIOORGANIC & MEDICINAL CHEMISTRY, PERGAMON, GB, vol. 8, no. 2, 1 February 2000 (2000-02-01), pages 413-426, XP009166338, ISSN: 0968-0896, DOI: 10.1016/S0968-0896(99)00302-8 [retrieved on 2000-02-23]
- JULIA QUINTON ET AL: "Reaction Discovery by Using a Sandwich Immunoassay", ANGEWANDTE CHEMIE. INTERNATIONAL EDITION, WILEY VCH VERLAG, WEINHEIM, vol. 51, no. 25, 18 June 2012 (2012-06-18) , pages 6144-6148, XP009166340, ISSN: 1433-7851, DOI: 10.1002/ANIE.201201451 [retrieved on 2012-05-04]
- LAVENTINE, DOMINIC M. ET AL: "Stereoselective synthesis by double reductive amination ring closure of novel aza-heteroannulated sugars", TETRAHEDRON , 65(24), 4766-4774 CODEN: TETRAB; ISSN: 0040-4020, 2009, XP026106656,

## Description

The present invention relates to a compound of formula (I) 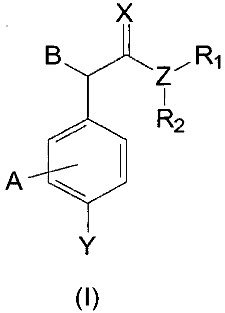 wherein X is NH; Y is OH; A is H or COOH; B is H, CH₂OH or CH₂NH₂; (a) ZR₁R₂ is isoquinolin-1-yl, said isoquinolin-1-yl being partially or fully hydrated in the N-containing ring; or given by formula (II) 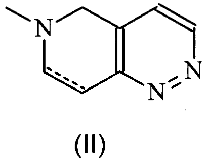 or (b) Z is O or N; R₁ is (i) C₁ to C₆ alkyl, preferably CH₃; C₂ to C₆ alkenyl; or C₂ to C₆ alkynyl; or (ii) arylalkyl or heteroarylalkyl, preferably phenylalkyl, alkyl in said arylalkyl or heteroarylalkyl being C₁ to C₄ alkyl, phenylalkyl preferably being CH₂C₆H₅; R₂ is (iii) absent in case of Z being O; or (iv) H in case of Z being N; with the proviso that 2-(4-hydroxyphenyl)-N'-methylethanimidamide and 2-(4-hydroxyphenyl)-N'-ethylethanimidamide are excluded.

As a consequence of an increasing number of resistances against established treatments, the indication area of malaria is characterised by a high need of novel approaches to treatment and prevention. In view of resistances against the previously successful treatments and drugs, nowadays combinations of agents comprising artemisinin are used in China, South-east-Asia and Africa. An example of such artemisinin-based combination therapy is Coartem®.

Myosins, as they occur in parasites of the *Apicomplexa* class are key molecules involved in at least two distinct processes of invasion as they occur during the life cycle of the parasite. The first invasion process is that of the *Apicomplexa* sporozoites which are transferred by the Anopheles mosquito and attack liver cells. In the liver merozoites develop which attack human erythrocytes, where further stages develop and proliferation occurs. Throughout the lifecycle motility of the parasites is conferred by myosins.

Agents which are targeted to these invasion processes and capable of inhibiting them effectively have a significant therapeutic potential. When developing new lead structures and potential active agents, particular attention has to be paid to specificity of the compounds for their target molecule such that biologic activity is confined to myosins of the parasites and the host organism is affected to the smallest extent possible.

An alkaloid isolated from *Tiliacora triandra* has been described to exhibit anti-malarial activity; see Pavanand et al. (1989). Radau et al. (1996) as well as Pachaly and Schäfer (1989) describe the synthesis of *Tiliacora* alkaloids. The *Tiliacora* alkaloid comprises a five membered polycyclic ring structure as well as a bi-phenyl moiety.
In view of the above described deficiencies of the established therapeutic approaches, the technical problem underlying the present invention can be seen in the provision of alternative or improved means and methods of treating or preventing malaria as well as other parasitic disorders. This technical problem is solved by the subject matter of the present claims.

Accordingly, the present invention provides a compound of formula (I) 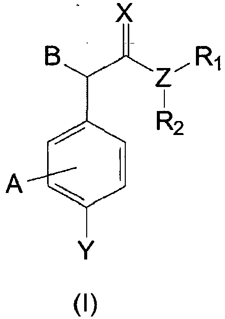 wherein X is NH; Y is OH; A is H or COOH; B is H, CH₂OH or CH₂NH₂; (a) ZR₁R₂ is isoquinolin-1-yl, said isoquinolin-1-yl being partially or fully hydrated in the N-containing ring; or given by formula (II) 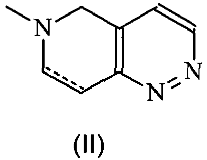 or (b) Z is O or N; R₁ is (i) C₁ to C₆ alkyl, preferably CH₃; C₂ to C₆ alkenyl; or C₂ to C₆ alkynyl; or (ii) arylalkyl or heteroarylalkyl, preferably phenylalkyl, alkyl in said arylalkyl or heteroarylalkyl being C₁ to C₄ alkyl, phenylalkyl preferably being CH₂C₆H₅; R₂ is (iii) absent in case of Z being O; or (iv) H in case of Z being N; with the proviso that 2-(4-hydroxyphenyl)-N'-methylethanimidamide and 2-(4-hydroxyphenyl)-N'-ethylethanimidamide are excluded.

Preferably, said C₁ to C₆ alkyl is C₁ to C₄ alkyl. Also preferred is that said alkyl is n-alkyl. Accordingly, particularly preferred is that C₁ to C₆ alkyl is selected from methyl, ethyl, n-propyl and n-butyl (in order of decreasing preference). Within C₂ to C₆ alkenyl and C₂ to C₆ alkynyl, preference is given to C₂ to C₄ alkenyl and C₂ to C₄ alkynyl, respectively. Preference is also given to unbranched forms as compared to branched forms. Particularly preferred alkenyls are ethenyl and 1-prop-2-enyl (allyl), expecially allyl. Preferred alkynyls are ethinyl and 1-prop-2-inyl.

Preferred aryl and heteroaryl moieties in said arylalkyl and heteroarylalkyl consist of one or two rings. Each ring preferably consists of five or six member atoms. In case of heteroaryl, preference is given to one member atom of said heteroaryl being a heteroatom. A preferred heteroatom is N. Further preferred heteroatoms are O and S.

It is understood that in said arylalkyl and heteroarylalkyl moiety, respectively, it is the alkyl moiety which comprises the valence connected to Z. Preferably, said alkyl moiety is C₁ to C₄ alkyl. Also preferred is that said alkyl moiety is n-alkyl, in particular n-propyl or n-butyl. Particularly preferred is that arylalkyl is phenylalkyl, in particular phenylmethyl (benzyl) or phenylethyl. Another preferred arylalkyl moiety is naphthylmethyl or naphtylethyl. A preferred heteroarylalkyl moiety is pyridinylalkyl, more specifically pyridinylmethyl or pyridinylethyl.

The compounds in accordance with the present invention may comprise one or more groups the protonation state of which is pH-dependent. It is understood that the representation as given herein includes all protonation states. For example, CHB-CNH-O includes CHB-CNH₂⁺-O and CHB-CNH-NH includes CHB-CNH₂⁺-NH. Similarly, it is understood that COOH includes COO⁻.

The present compounds are novel small molecules which are capable of inhibiting, preferably specifically inhibiting, myosins, preferably class 14 myosins, and more preferred myosins of the *Apicomplexa* class of parasites.

Generally speaking, it is preferred that said compound of formula (I) inhibits a myosin. The inhibition of a myosin can be determined with assays well known in the art and at the skilled person's disposal. For example, the ATPase activity of myosin may be assayed in presence and absence of a compound of formula (I). For example, the ATPase activity to be assayed may be the basal myosin ATPase activity or the ATPase activity of myosin in its actin-activated state. A further suitable assay is an in vitro motility assay which monitors the activity of myosin in the presence of ATP and actin. Also in such an assay, myosin activity (in this case motility) in presence and absence of a compound of formula (I) is compared.

Preferably, said compound is provided in isolated form. If it is provided as a composition or as a pharmaceutical composition (see below), it is preferred that said composition comprises said compound of formula (I), or more than one compound of formula (I) as the only active agent(s). Deliberately envisaged, however, is also that such formulation or pharmaceutical composition comprises, in addition to one or more compounds of formula (I), further pharmaceutically active agents, for example pharmaceutically active agents useful in the treatment of malaria or any parasitic disorder as disclosed herein.

Turning to the specific medical indication malaria which is a particularly preferred disease to be targeted by the compounds according to the present invention, it is of note that, as mentioned in the background section herein above, myosins, especially class 14 myosins, are of importance at various development stages of the causative parasite.

In a further preferred embodiment of the compound of the invention, A is H.

In a further preferred embodiment of the compound of the invention, B is H.

In a further preferred embodiment of the compound of the invention, ZR₁R₂ is isoquinolin-1-yl, said isoquinolin-1-yl being at least partially hydrated in the N-containing ring, preferably fully hydrated in the N-containing ring. Said partially and fully hydrated forms of isoquinolin-1-yl are dihydro- and tetrahydro-isoquinolin-1-yl. Preferred compounds of the invention comprising a tetrahydro-isoquinolin group are V, VI and VIII as disclosed further below.

In a preferred embodiment of the compound of formula (I), X is NH and Z is N.

In a further preferred embodiment of the compound of formula (I), X is NH, A is COOH, B is H, CH₂OH or CH₂NH₂, preferably H, and ZR₁R₂ is isoquinolin-1-yl, said isoquinolin-1-yl being at least partially hydrated in the N-containing ring, preferably fully hydrated in the N-containing ring. Accordingly, particularly preferred is that X is NH, A is COOH, B is H and ZR₁R₂ is tetrahydro-isoquinolin-1-yl, said tetrahydro-isoquinolin-1-yl being fully hydrated in the N-containing ring.

In a further preferred embodiment of the compound of the invention, A is COOH, said COOH being in ortho-position with regard to Y. Said preferred ortho-position is illustrated the particularly preferred compounds (V), (VII) and (VIII) as disclosed further below.

In a further preferred embodiment of the compound of the invention, the carbon atom bearing the group B is in the (S) configuration. This preferred stereochemistry is illustrated by the particularly preferred compounds (V) to (VIII) as disclosed further below. Having said that, it is expected that also the (R) configuration will exert significant activity albeit generally lower than the (S) configuration of a given compound.

Particularly preferred are compounds of formula (IV) to (VIII)

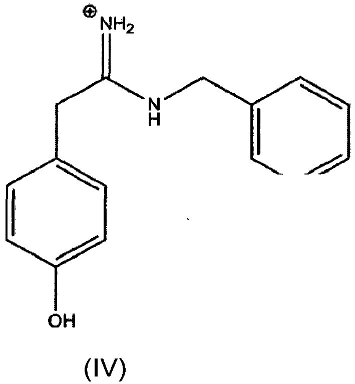

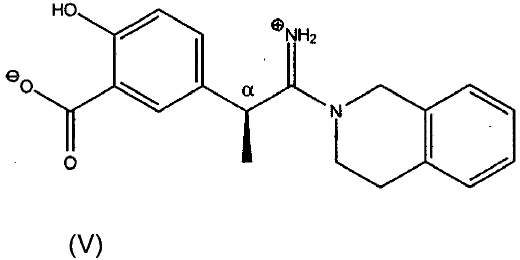

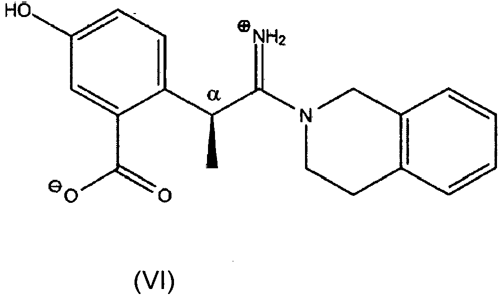

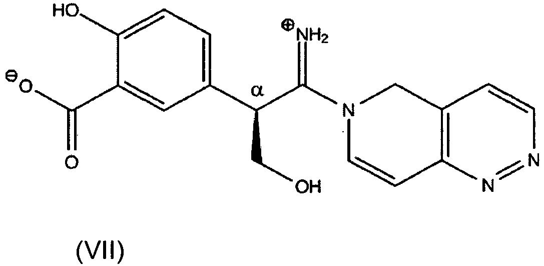

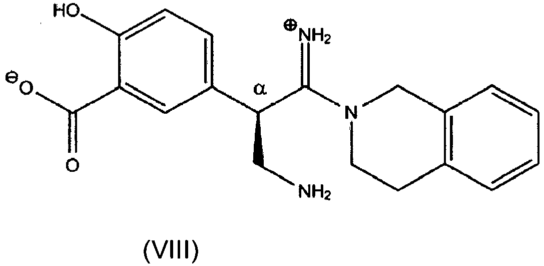

The present invention furthermore provides a pharmaceutical composition comprising one or more compounds according to the invention.

In a preferred embodiment of the pharmaceutical composition according to the invention, one or more compounds of the invention are the only pharmaceutically active agents comprised in said pharmaceutical composition.

The pharmaceutical compositions disclosed herein can be administered to the subject at a suitable dose. Administration of the suitable compositions may be effected by different ways, e.g., by intravenous, intraperitoneal, subcutaneous, as well as transdermal administration.

More specifically, the pharmaceutical compositions may be administered orally, parenterally, such as subcutaneously, intravenously, intramuscularly, intraperitoneally, intrathecally, transdermally, transmucosally, subdurally, nasal, locally or topically via iontopheresis, sublingually, by inhalation spray, aerosol or rectally and the like in dosage unit formulations optionally comprising conventional pharmaceutically acceptable excipients.

The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently.

Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like. Furthermore, the pharmaceutical composition described herein may comprise further agents depending on the intended use of the pharmaceutical composition.

Pharmaceutically useful excipients that may be used in the formulation may comprise carriers, vehicles, diluents, solvents such as monohydric alcohols such as ethanol, isopropanol and polyhydric alcohols such as glycols and edible oils such as soybean oil, coconut oil, olive oil, safflower oil cottonseed oil, oily esters such as ethyl oleate, isopropyl myristate; binders, adjuvants, solubilizers, thickening agents, stabilizers, disintergrants, glidants, lubricating agents, buffering agents, emulsifiers, wetting agents, suspending agents, sweetening agents, colourants, flavours, coating agents, preservatives, antioxidants, processing agents, drug delivery modifiers and enhancers such as calcium phosphate, magnesium state, talc, monosaccharides, disaccharides, starch, gelatine, cellulose, methylcellulose, sodium carboxymethyl cellulose, dextrose, hydroxypropyl-ß-cyclodextrin, polyvinylpyrrolidone, low melting waxes, and/or ion exchange resins.

Other suitable pharmaceutically acceptable excipients are described in Remington's Pharmaceutical Sciences, 15th Ed., Mack Publishing Co., New Jersey (1991).

Dosage forms for oral administration include tablets, capsules, lozenges, pills, wafers, granules, oral liquids such as syrups, suspensions, solutions, emulsions, powder for reconstitution.

Dosage forms for local/topical administration comprise insufflations, aerosols, metered aerosols, transdermal therapeutic systems, medicated patches, rectal suppositories, and/or ovula.

For the purpose of the present invention, a therapeutically effective dosage of the recited agents may preferably be from about 1 to 1000 mg/day, preferably from about 5 to about 50 mg/day, and most preferably from about 10 to about 250 mg/day, which may be administered in one or multiple doses.

It will be appreciated, however, that specific dose level of the compounds of the invention for any particular patient will depend on a variety of factors such as age, sex, body weight, general health condition, diet, individual response of the patient to be treated time of administration, severity of the disease to be treated, the activity of particular compound applied, dosage form, mode of application and concomitant medication. The therapeutically effective amount for a given situation will readily be determined by routine experimentation and is within the skills and judgement of the ordinary clinician or physician.

In a further aspect, the present invention provides one or more compound(s) of formula (I) or (III) as given below or of any one of formulae (IV) to (VIII) as shown above for use in treating or preventing a disease caused by a parasite of the group of *Apicomplexa:* 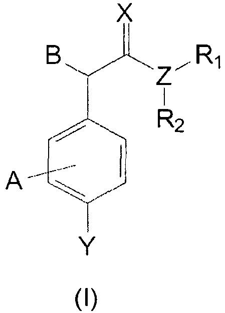 wherein X is NH; Y is OH; A is H or COOH; B is H, CH₂OH or CH₂NH₂; (a) ZR₁R₂ is isoquinolin-1-yl, said isoquinolin-1-yl being partially or fully hydrated in the N-containing ring; or given by formula (II) 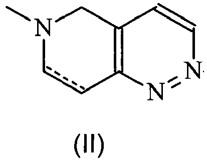 or (b) Z is O or N; R₁ is (i) C₁ to C₆ alkyl, preferably CH₃; C₂ to C₆ alkenyl; or C₂ to C₆ alkynyl; or (ii) arylalkyl or heteroarylalkyl, preferably phenylalkyl, alkyl in said arylalkyl or heteroarylalkyl being C₁ to C₄ alkyl, phenylalkyl preferably being CH₂C₆H₅; R₂ is (iii) absent in case of Z being O; or (iv) H in case of Z being N

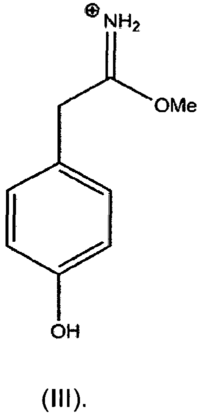

*Apicomplexa* are a group of eukaryotic protists. They are capable of forming spores, and they are exclusively parasitic. When present in animals, they give rise to characteristic symptoms of parasitic diseases. Specific diseases associated with the presence of the parasites of the group of *Apicomplexa* are detailed further below. The compounds according to the invention are useful for both treating as well as for preventing a disease caused by a parasite of the group of *Apicomplexa.*

In a preferred embodiment, one or more compound(s) are the only pharmaceutically active agents to be used in said treating or preventing.

In further preferred embodiments, said disease and said parasite, respectively, are (a) *Plasmodium* and malaria; (b) *Toxoplasma gondii* and toxoplasmosis; (c) *Eimeria* and coccidiosis; (d) *Isospora* and isosporiasis/coccidiosis; (e) *Babesia* and babesiosis; (f) *Cyclospora* and cyclosporiasis; (g) *Cryptosporidium* and cryptosporidiosis; (h) *Theileria* and theileriosis; (i) *Neospora* and neosporosis; (j) *Sarcocystis*/*Hoareosporidium* and sarcocystiosis.

Particular preferred is that said parasite is *Plasmodium falciparum.*

Preferably, said myosin is a class 14 myosin. Class 14 myosins are produced by apicomplexan parasites like *Toxoplasma* or the malaria parasite *Plasmodium* but not by host cells. Parasites belonging to the apicomplexa that infect animals or humans include also the genera *Eimeria, Isospora, Cyclospora, Babesia, Cryptosporidium, Theileria* and *Sarcocystis.* Apicomplexa move and actively penetrate host cells relying on an actomyosin-dependent mode of motion, which is generally referred to as gliding motility. Members of class-14 are generally small in size and display low sequence similarity of around 30 % with the motor domains of conventional myosins. They lack a conventional neck region, as indicated by the absence of proper IQ motifs. In addition to their role in the motility and invasion, class-14 myosin C from *Toxoplasma gondii* is involved in cell division and replication of the parasites.

Particularly preferred is that said myosin is *Plasmodium falciparum* myosin A.

The figures show:
**Figure 1****:** IC₅₀ values of compounds of the invention determined using Dictyostelium discoideum (Dd) Myosin -1 b.
**Figure 2****:** Sliding speed in *in vitro* motility assay (see Example 2). "-" is the negative control (no myosin inhibitors). Numbers indicate compounds according to the invention; see also Example 3. In each case the three bars indicate from left to right: Dd Myosin -1b, *Homo sapiens* (Hs) non-muscle Myosin-2a, and Hs Myosin-7.

The examples illustrate the invention.

### Example 1:

### Chemical synthesis

Scheme 1 below provides an outline of the synthesis route for compounds of the invention. Compounds **17** and **19** or also referred to as "BIP13" and "BIP14", respectively.

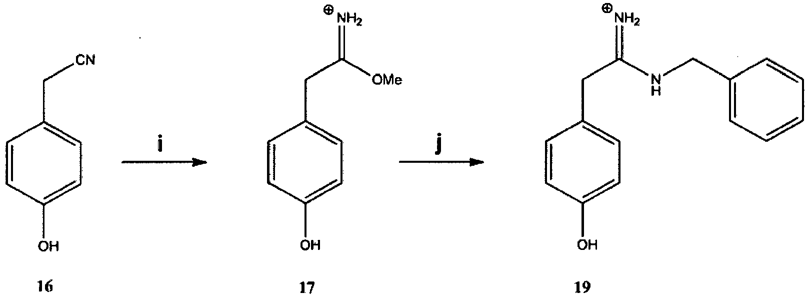

### Methyl-2-(4-hydroxyphenyl)acetimidate·hydrochloride (BIP13) (i):

2-(4-Hydroxyphenyl)acetonitrile (100 mg, 0.75 mmol) was dissolved in absolute MeOH (5mL) under nitrogen atmosphere and cooled to -20 °C. Acetylchloride (1.78 mL, 25 mmol) was added and the mixture was allowed to warm slowly to room temperature and stirred overnight. After removing the solvent with the rotation evaporator and under high vacuum, the product was received as a colorless solid. **BIP13** (149.9 mg, 0.74 mmol, 99 %).

**¹H-NMR (400 MHz, CH₃OD):** *δ* = ppm 7.18 (d, *³J* = 8.77 Hz, 2H), 6.80 (d, *³J* = 8.77 Hz, 2H), 4.90 (s, 3H), 3.77 (s, 2H).
**¹³C**-**NMR (100 MHz, CH₃OD):** *δ* = ppm 173.2 (1 C), 156.2 (1 C), 129.9 (2C), 128.8 (1 C), 114.9 (2C), 51.0 (1 C), 39.5 (1 C).

### N-Benzyl-2-(4-hydroxyphenyl)acetimidamide-hydrochloride (BIP14) (j):

To a mixture of **BIP13** (35 mg, 0.17 mmol) in absolute MeOH (2 mL) benzylamine (20.4 µL, 0.19 mmol) was added. The mixture was refluxed for 12 hours, subsequently concentrated with the rotation evaporator and precipitated with Et₂O. The product was received as a pale solid. **BIP14** (19.6 mg, 0.071 mmol, 41 %).

**¹H-NMR (400 MHz, CH₃OD):** *δ* = ppm 7.37 (m, 2H), 7.35 (m, 3H), 7.19 (d, *³J* = 8.79 Hz, 2H), 6.81 (d, *³J* = 8.79 Hz, 2H), 3.82 (s, 2H), 3.79 (s, 2H).

### Example 2:

### Activity tests

### ATPase assay

In this assay, the ATPase activity of *Dictyostelium discoideum* myosin-1b is determined, preferably at a ligand concentration of 25µM. Furthermore, the IC₅₀ concentration has been determined by measuring ATPase activity as a function of inhibitor concentration. Alternatively *Dictyostelium discoideum* myosin-2 can be used.

### Growth assay

Growth assays have been performed using merozoites of *Plasmodium falciparum* strains 3D7 and FCA3. These growth assays are known in the art as "SYBR Green" assays; see, for example, Johnson et al. (2007). In this assay, the life cycle of the merozoites (consisting of invasion of human erythrocytes, asexual replication, release from erythrocytes and further invasion) is determined in the presence of different concentrations of compounds according to the invention.

### Gliding motility assay

This assay provides for determining the effect of compounds of the invention on sporozoites. The assay involves semiautomatic *in vitro* imaging of a plurality of sporozoites at a given time; see, for example, Hegge et al. (2009). Sporozoites are labeled with green fluorescent protein (GFP) and exhibit counter clockwise circular movement under normal conditions. Upon the addition of compounds according to the present invention, the number of sporozoites moving in a circular manner is reduced; instead waving or immobile sporozoites are observed. Measurements have been performed at a concentration of 100µM.

### In vitro motility assay

The activity of the small molecule compounds on the myosin motor function was studied using an in vitro motility assay (Kron (1986); Anson (1996)). The sliding velocities of fluorescently labeled actin filaments over myosin motor domain constructs, which were fixed to a glass surface, were analyzed by fluorescence microscopy.

Taken together, the evidence provided above shows that compounds according to the invention are active in an *in vitro* assay (ATPase assay) and furthermore against the two stages of *Plasmodium,* which stages are merozoites and sporozoites.

### Example 3:

### Characterization of compounds in terms of their activity

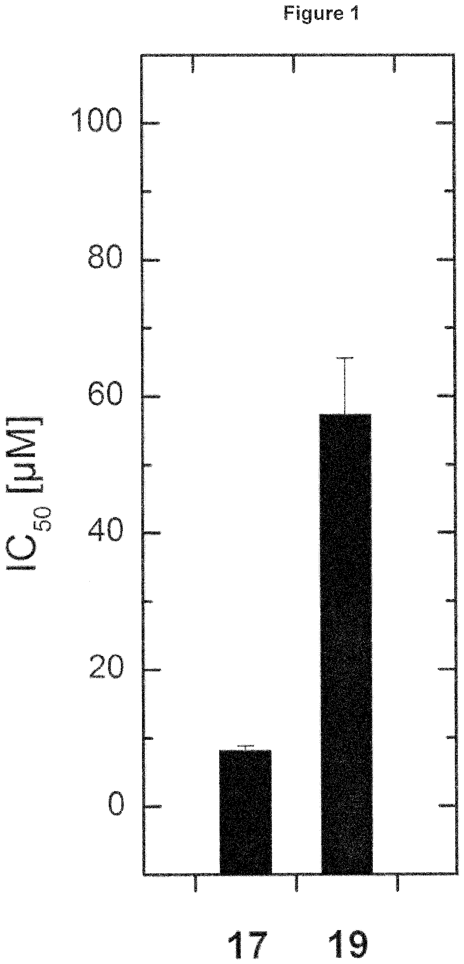

Actin-activated steady state ATPase experiments were performed following the absorbance change during NADH oxidation in a PK/LDH-coupled ATPase assay at 340 nm excitation (Trentham (1972); Furch (1998)). The photometric steady state ATPase assay was applied to determine the potency of the small molecule compounds to inhibit the enzymatic activity of myosin. Dd myosin-1b was used as a model system for the target protein (Pf myosin A). All experiments were carried out in the presence of 30 µM F-actin and the corresponding half-maximal inhibitory concentrations (IC₅₀) were determined by nonlinear regression. Methyl-2-(4-hydroxyphenyl)acetimidate·hydrochloride **(BIP13;** compound **17)** showed an IC₅₀ in the low micromolar range (8.07 ± 0.8 µM), while N-benzyl-2-(4-hydroxyphenyl)acetimidamide·hydrochloride **(BIP14;** compound **19)** exhibited a moderate IC₅₀ of 57.27 ± 8.3 µM (Fig. 1).

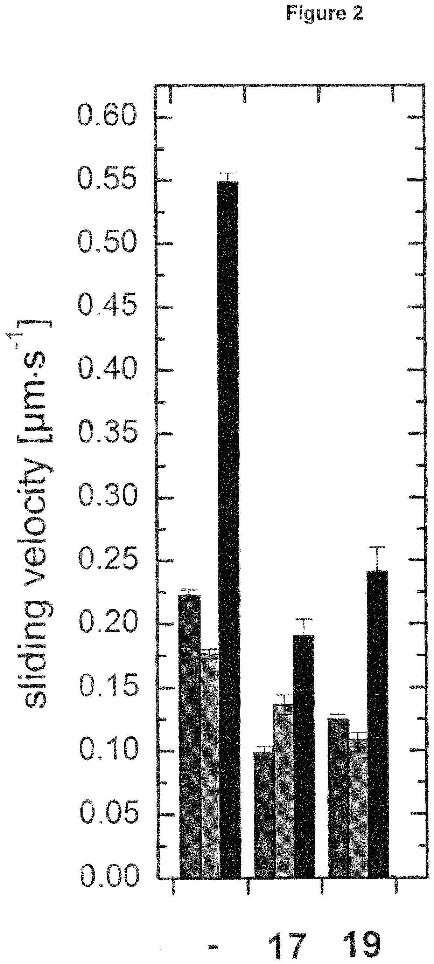

The activity of the small molecule compounds on the myosin motor function was studied using an in vitro motility assay (Kron (1986); Anson (1996)). The sliding velocities of fluorescently labeled actin filaments over myosin motor domain constructs, which were fixed to a glass surface, were analyzed by fluorescence microscopy. Three different myosin isoforms were tested: *Dd* myosin-1b served as a model system for the target protein, and the two human isoforms *Hs* nonmuscle myosin-2a and Hs myosin-7. The lead structures resulted in a significant reduction in the sliding velocity of the actin filaments with different profiles for the three myosin isoforms (Fig. 2).

### Further References

Radau, G. et al. (1996), Zur Synthese von Tiliacora-Alkaloiden-III: Synthese von Biarylen durch Ullmann-Kupplung, Tetrahedron, 52(47), 14735-14744.
Pachaly, P. and Schäfer, M. (1988), Darstellung des unsymmetrischen Biphenyl-Schwanzteils, Arch. Pharm. (Weinheim), 322, 483-487.
Pavanand, K. et al. (1989), Antimalarial activity of Tiliacora triandra Diels against Plasmodium falciparum in vitro, Phytotherapy Research, 3(5), 215-217.
Johnson et al. (2007), Assessment of Continued Validation of the Malaria SYBR Green I-Based Fluorescence Assay for Use in Malaria Drug Screening, Antimicrobial Agents and Chemotherapy, 51, 1926-1933.
Hegge et al. (2009), Automated classification of Plasmodium sporozoite movement patterns reveals a shift towards productive motility during salivary gland infection, Biotechnological Journal*.*
Kron et al. (1986), PNAS, 83, 6272-6276
Anson et al. (1996), EMBO J., 15, 6069-6074
Trentham et al. (1972), Biochem. J., 126, 635-644
Furch et al. (1998), Biochemistry, 37, 6317-6326

## Claims

1. A compound of formula (I) 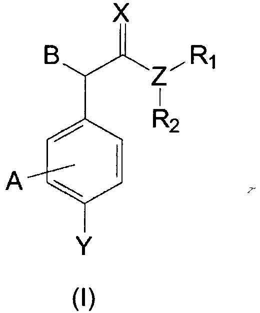 wherein
X is NH;
Y is OH;
A is H or COOH;
B is H, CH₂OH or CH₂NH₂;
(a) ZR₁R₂ is isoquinolin-1-yl, said isoquinolin-1-yl being partially or fully hydrated in the N-containing ring; or given by formula (II) 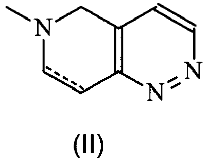 or
(b)
Z is O or N;
R₁ is
(i) C₁ to C₆ alkyl, preferably CH₃; C₂ to C₆ alkenyl; or C₂ to C₆ alkynyl; or
(ii) arylalkyl or heteroarylalkyl, preferably phenylalkyl, alkyl in said arylalkyl or heteroarylalkyl being C₁ to C₄ alkyl, phenylalkyl preferably being CH₂C₆H₅;
R₂ is
(iii) absent in case of Z being O; or
(iv) H in case of Z being N;
with the proviso that 2-(4-hydroxyphenyl)-N'-methylethanimidamide and 2-(4-hydroxyphenyl)-N'-ethylethanimidamide are excluded.

2. The compound of claim 1, wherein A is H.

3. The compound of any one of the preceding claims, wherein B is H.

4. The compound of any one of the preceding claims, wherein ZR₁R₂ is isoquinolin-1-yl, said isoquinolin-1-yl being at least partially hydrated in the N-containing ring, preferably fully hydrated in the N-containing ring.

5. The compound of claim 1, wherein Z is N.

6. The compound of claim 1, wherein X is NH, A is COOH, B is H, CH₂OH or CH₂NH₂, preferably H, and ZR₁R₂ is isoquinolin-1-yl, said isoquinolin-1-yl being at least partially hydrated in the N-containing ring, preferably fully hydrated in the N-containing ring.

7. The compound of claim 1 or 6, wherein A is COOH, said COOH being in ortho-position with regard to Y.

8. The compound of any one of the preceding claims, wherein the carbon atom bearing the group B is in the (S) configuration.

9. A compound selected from the compounds of formulae (IV) to (VIII):

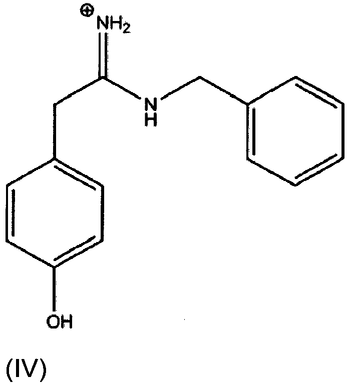

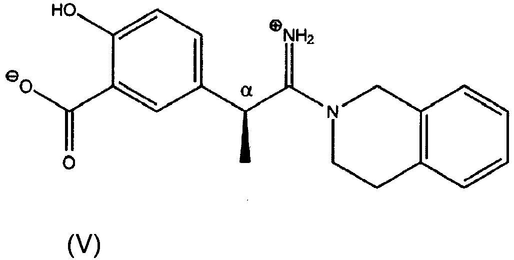

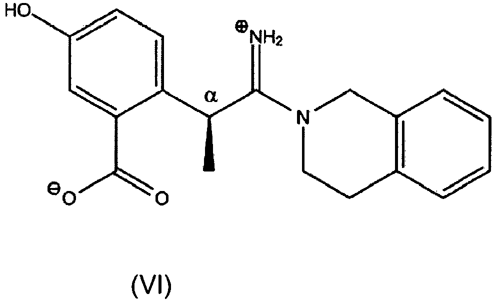

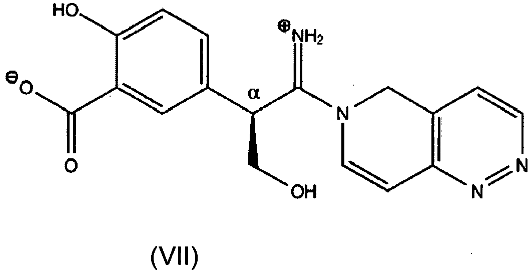

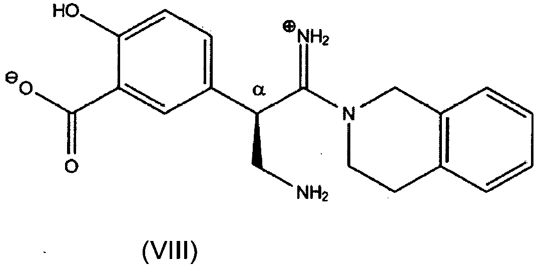

10. A pharmaceutical composition comprising one or more compounds as defined in any one of the preceding claims.

11. The pharmaceutical composition of claim 10, wherein said one or more compounds are the only pharmaceutically active agents comprised in said pharmaceutical composition.

12. One or more compound(s) of formula (I) or (III) as given below or of any one of formulae (IV) to (VIII) as defined in claim 9 for use in treating or preventing a disease caused by a parasite of the group of *Apicomplexa:* 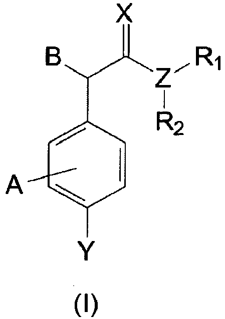 wherein
X is NH;
Y is OH;
A is H or COOH;
B is H, CH₂OH or CH₂NH₂; (a
(a) ZR₁R₂ is isoquinolin-1-yl, said isoquinolin-1-yl being partially or fully hydrated in the N-containing ring; or given by formula (II) 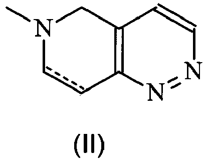 or
(b)
Z is O or N;
R₁ is
(i) C₁ to C₆ alkyl, preferably CH₃; C₂ to C₆ alkenyl; or C₂ to C₆ alkynyl; or
(ii) arylalkyl or heteroarylalkyl, preferably phenylalkyl, alkyl in said arylalkyl or heteroarylalkyl being C₁ to C₄ alkyl, phenylalkyl preferably being CH₂C₆H₅;
R₂ is
(iii) absent in case of Z being O; or
(iv) H in case of Z being N;
or

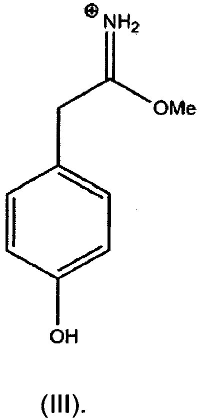

13. The compound(s) for use of claim 12, wherein said one or more compound(s) are the only pharmaceutically active agents to be used in said treating or preventing.

14. The compound(s) for use of claim 12 or 13, wherein said disease and said parasite, respectively, are
(a) *Plasmodium* and malaria, said parasite preferably being *Plasmodium falciparum*;
(b) *Toxoplasma gondii* and toxoplasmosis;
(c) *Eimeria* and coccidiosis;
(d) *Isospora* and isosporiasis/coccidiosis;
(e) *Babesia* and babesiosis;
(f) *Cyclospora* and cyclosporiasis;
(g) *Cryptosporidium* and cryptosporidiosis;
(h) *Theileria* and theileriosis;
(i) *Neospora* and neosporosis;
(j) *Sarcocystis*/*Hoareosporidium* and sarcocystiosis.

15. The compound(s) for use of any one of claims 12 to 14, wherein A is H.

16. The compound(s) for use of any one of claims 12 to 15, wherein B is H.

17. The compound(s) for use of any one of claims 12 to 16, wherein ZR₁R₂ is isoquinolin-1-yl, said isoquinolin-1-yl being at least partially hydrated in the N-containing ring, preferably fully hydrated in the N-containing ring.

18. The compound(s) for use of any one of claims 12 to 14, wherein Z is N.

19. The compound(s) for use of any one of claims 12 to 14, wherein X is NH, A is COOH, B is H, CH₂OH or CH₂NH₂, preferably H, and ZR₁R₂ is isoquinolin-1-yl, said isoquinolin-1-yl being at least partially hydrated in the N-containing ring, preferably fully hydrated in the N-containing ring.

20. The compound(s) for use of any one of claims 12 to 14 or 19, wherein A is COOH, said COOH being in ortho-position with regard to Y.

21. The compound(s) for use of any one of claims 12 to 20, wherein the carbon atom bearing the group B is in the (S) configuration.

## Patentansprüche

1. Verbindung der Formel (I) 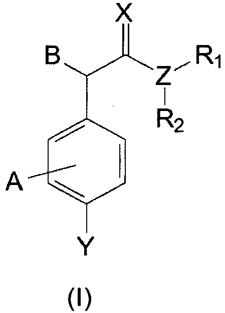 wobei
X NH ist;
Y OH ist;
A H oder COOH ist;
B H, CH₂OH oder CH₂NH₂ ist;
(a) ZR₁R₂ Isoquinolin-1-yl ist, wobei Isoquinolin-1-yl im N-enthaltenden Ring teilweise oder vollständig hydriert ist; oder durch die Formel (II) gegeben ist 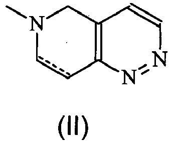 oder
(b)
Z O oder N ist;
R₁
(i) C₁ bis C₆ Alkyl, vorzugsweise CH₃; C₂ bis C₆ Alkenyl; oder C₂ bis C₆ Alkynyl ist; oder
(ii) Arylalkyl oder Heteroarylalkyl ist, vorzugsweise Phenylalkyl, wobei Alkyl in Arylalkyl oder Heteroarylalkyl C₁ bis C₄ Alkyl ist, und Phenylalkyl vorzugsweise CH₂C₆H₅ ist;
R₂
(iii) abwesend ist im Falle, dass Z O ist; oder
(iv) H ist im Falle, dass Z N ist;
mit der Maßgabe, dass 2-(4-Hydroxyphenyl)-N'-Methylethanimidamid und 2-(4-Hydroxyphenyl)-N'-ethylethanimidamid ausgeschlossen sind.

2. Verbindung nach Anspruch 1, wobei A H ist.

3. Verbindung nach einem der vorangegangenen Ansprüche, wobei B H ist.

4. Verbindung nach einem der vorangegangenen Ansprüche, wobei ZR₁R₂ Isoquinolin-1-yl ist, wobei Isoquinolin-1-yl im N-enthaltenden Ring zumindest teilweise hydriert, vorzugsweise vollständig hydriert ist.

5. Verbindung nach Anspruch 1, wobei Z N ist.

6. Verbindung nach Anspruch 1, wobei X NH, A COOH, B H, CH₂OH oder CH₂NH₂, vorzugweise H, und ZR₁R₂ Isoquinolin-1-yl ist, wobei Isoquinolin-1-yl im N-enthaltenden Ring zumindest teilweise hydriert, vorzugsweise vollständig hydriert ist.

7. Verbindung nach Anspruch 1 oder 6, wobei A COOH ist, wobei COOH in Orthostellung hinsichtlich Y ist.

8. Verbindung nach einem der vorangegangen Ansprüche, wobei das Kohlenstoffatom, das die Gruppe B trägt, in der (S) Konfiguration ist.

9. Verbindung ausgewählt aus den Verbindungen der Formeln (IV) bis (VIII):

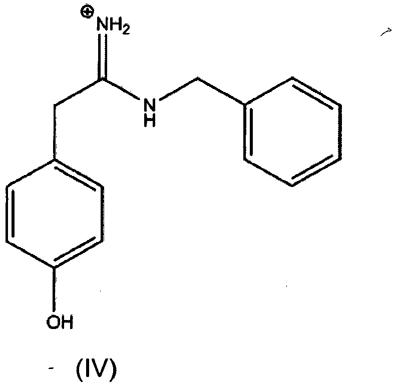

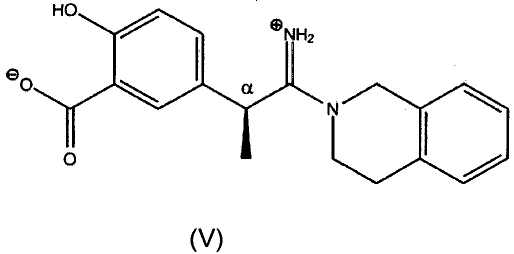

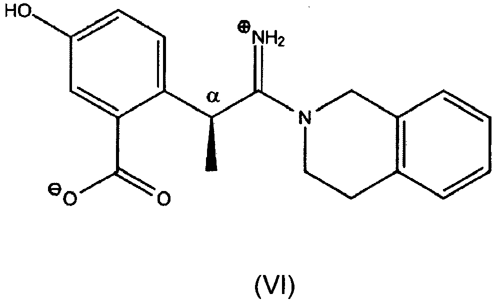

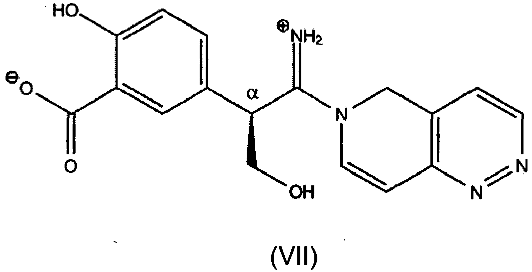

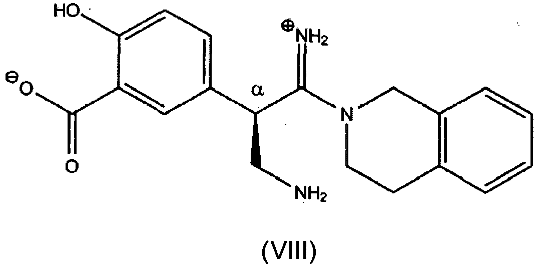

10. Pharmazeutische Zusammensetzung umfassend eine oder mehrere in einem der vorangegangen Ansprüche definierten Verbindungen.

11. Pharmazeutische Zusammensetzung nach Anspruch 10, wobei die eine Verbindung oder die mehreren Verbindungen die einzigen pharmazeutisch aktiven Agenzien sind, die in der pharmazeutischen Zusammensetzung enthalten sind.

12. Eine oder mehrere Verbindung(en) der Formel (I) oder (III) wie im Folgenden dargestellt oder in einer der Formeln (IV) bis (VIII) wie in Anspruch 9 definiert, für die Verwendung zum Behandeln oder Vorbeugen einer Krankheit, die von einem Parasiten der Gruppe der *Apicomplexa* verursacht ist: 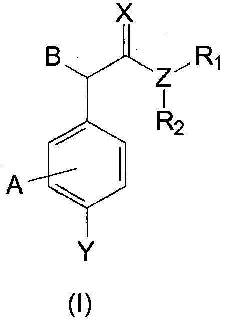 wobei
X NH ist;
Y OH ist;
A H oder COOH ist;
B H, CH₂OH oder CH₂NH₂ ist;
(a) ZR₁R₂ Isoquinolin-1-yl ist, wobei Isoquinolin-1-yl im N-enthaltenden Ring teilweise oder vollständig hydriert ist; oder durch die Formel (II) gegeben ist 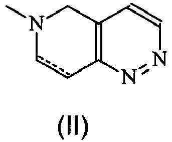 oder
(b)
Z O oder N ist;
R₁
(i) C₁ bis C₆ Alkyl, vorzugsweise CH₃; C₂ bis C₆ Alkenyl; oder C₂ bis C₆ Alkynyl ist; oder
(ii) Arylalkyl oder Heteroarylalkyl ist, vorzugsweise Phenylalkyl, wobei Alkyl in Arylalkyl oder Heteroarylalkyl C₁ bis C₄ Alkyl ist, und Phenylalkyl vorzugsweise CH₂C₆H₅ ist;
R₂
(iii) abwesend ist im Falle, dass Z O ist; oder
(iv) H ist im Falle, dass Z N ist;
oder

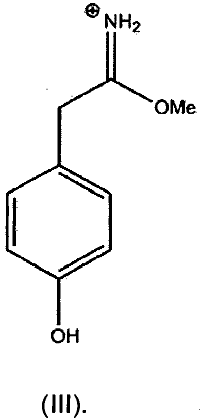

13. Verbindung(en) für die Verwendung nach Anspruch 12, wobei die eine oder die mehreren Verbindung(en) die einzigen pharmazeutisch aktiven Agenzien sind, die für das Behandeln oder Vorbeugen zu verwenden sind.

14. Verbindung(en) für die Verwendung nach Anspruch 12 oder 13, wobei die Krankheit und der Parasit jeweils die Folgenden sind
(a) *Plasmodium* und Malaria, wobei der Parasit vorzugsweise *Plasmodium falciparum* ist;
(b) *Toxoplasma gondii* und Toxoplasmose;
(c) *Eimeria* und Kokzidiose;
(d) *Isospora* und Isosporiasis/Kokzidiose;
(e) *Babesia* und Babesiose;
(f) *Cyclospora* und Zyclosporiasis;
(g) *Cryptosporidium* und Kryptosporidiose;
(h) *Theileria* und Theileriose;
(i) *Neospora* und Neosporose;
(j) *Sarcocystis*/*Hoareosporidium* und Sarcozystose.

15. Verbindung(en) für die Verwendung nach einem der Ansprüche 12 bis 14, wobei A H ist.

16. Verbindung(en) für die Verwendung nach einem der Ansprüche 12 bis 15, wobei B H ist.

17. Verbindung(en) für die Verwendung nach einem der Ansprüche 12 bis 16, wobei ZR₁R₂ Isoquinolin-1-yl ist, wobei Isoquinolin-1-yl im N-enthaltenden Ring zumindest teilweise hydriert, vorzugsweise vollständig hydriert ist.

18. Verbindung(en) für die Verwendung nach einem der Ansprüche 12 bis 14, wobei Z N ist.

19. Verbindung(en) für die Verwendung nach einem der Ansprüche 12 bis 14, wobei X NH, A COOH, B H, CH₂OH oder CH₂NH₂, vorzugweise H, und ZR₁R₂ Isoquinolin-1-yl ist, wobei Isoquinolin-1-yl im N-enthaltenden Ring zumindest teilweise hydriert, vorzugsweise vollständig hydriert ist.

20. Verbindung(en) für die Verwendung nach einem der Ansprüche 12 bis 14 oder 19, wobei A COOH ist, wobei COOH in Orthostellung hinsichtlich Y ist.

21. Verbindung(en) für die Verwendung nach einem der Ansprüche 12 bis 20, wobei das Kohlenstoffatom, das die Gruppe B trägt, in der (S) Konfiguration ist.

## Revendications

1. Composé de formule (I) 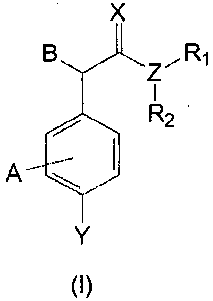 dans laquelle
X est NH ;
Y est OH ;
A est H ou COOH ;
B est H, CH₂OH ou CH₂NH₂ ;
(a) ZR₁R₂ est un groupe isoquinolin-1-yle, ledit groupe isoquinolin-1-yle étant partiellement ou totalement hydraté dans le cycle contenant N ; ou donné par la formule (II) 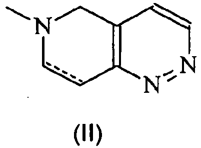 ou
(b)
Z est O ou N ;
R₁ est
(i) un groupe alkyle en C₁ à C₆, de préférence CH₃ ; un groupe alcényle en C₂ à C₆ ; ou un groupe alcynyle en C₂ à C₆ ; ou
(ii) un groupe arylalkyle ou hétéroarylalkyle, de préférence phénylalkyle, le groupe alkyle dans ledit groupe arylalkyle ou hétéroarylalkyle étant un groupe alkyle en C₁ à C₄, et le groupe phénylalkyle étant de préférence CH₂C₆H₅ ;
R₂ est
(iii) absent dans le cas où Z est O ; ou
(iv) H dans le cas où Z est N ;
à condition que le 2-(4-hydroxyphényl)-N'-méthyléthanimidamide et le 2-(4-hydroxyphényl)-N'-éthyléthanimidamide soient exclus.

2. Composé selon la revendication 1, dans lequel A est H.

3. Composé selon l'une quelconque des revendications précédentes, dans lequel B est H.

4. Composé selon l'une quelconque des revendications précédentes, dans lequel ZR₁R₂ est un groupe isoquinolin-1-yle, ledit groupe isoquinolin-1-yle étant au moins partiellement hydraté dans le cycle contenant N, de préférence totalement hydraté dans le cycle contenant N.

5. Composé selon la revendication 1, dans lequel Z est N.

6. Composé selon la revendication 1, dans lequel X est NH, A est COOH, B est H, CH₂OH ou CH₂NH₂, de préférence H, et ZR₁R₂ est un groupe isoquinolin-1-yle, ledit groupe isoquinolin-1-yle étant au moins partiellement hydraté dans le cycle contenant N, de préférence totalement hydraté dans le cycle contenant N.

7. Composé selon la revendication 1 ou 6, dans lequel A est COOH, ledit COOH étant en position ortho par rapport à Y.

8. Composé selon l'une quelconque des revendications précédentes dans lequel l'atome de carbone portant le groupe B est de configuration (S).

9. Composé choisi parmi les composés de formules (IV) à (VIII) :

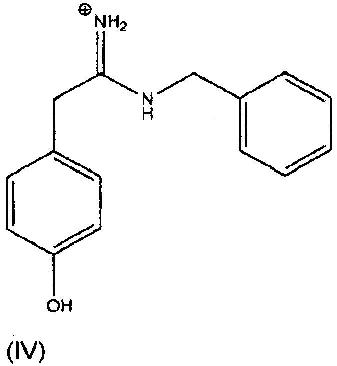

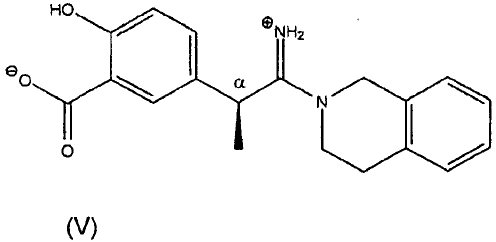

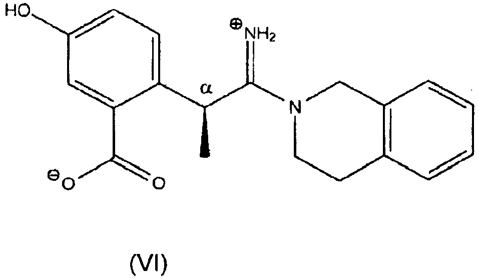

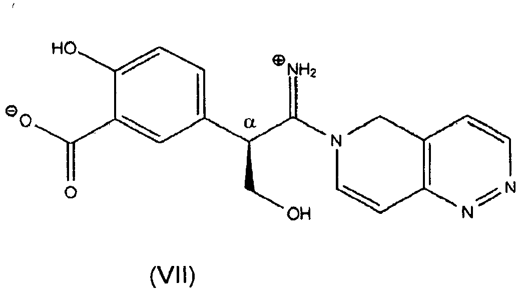

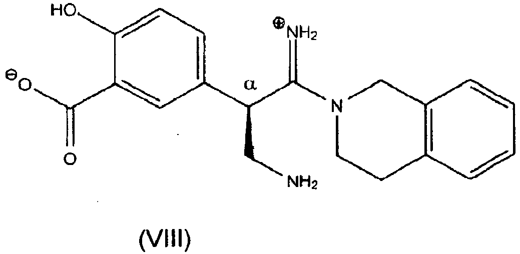

10. Composition pharmaceutique comprenant un ou plusieurs composés tels que définis dans l'une quelconque des revendications précédentes.

11. Composition pharmaceutique selon la revendication 10, dans laquelle ledit un composé ou lesdits plusieurs composés sont les seuls agents pharmaceutiquement actifs compris dans ladite composition pharmaceutique.

12. Un ou plusieurs composés de formule (I) ou (III) telle qu'indiquée ci-dessous ou selon l'une quelconque des formules (IV) à (VIII) telles que définies dans la revendication 9 pour une utilisation dans le traitement ou la prévention d'une maladie causée par un parasite du groupe des *Apicomplexa :* 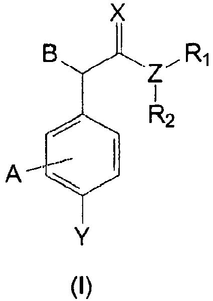 dans laquelle
X est NH ;
Y est OH ;
A est H ou COOH ;
B est H, CH₂OH ou CH₂NH₂ ;
(a) ZR₁R₂ est un groupe isoquinolin-1-yle, ledit groupe isoquinolin-1-yle étant partiellement ou totalement hydraté dans le cycle contenant N ; ou donné par la formule (II) 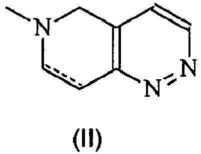 ou
(b)
Z est O ou N ;
R₁ est
(i) un groupe alkyle en C₁ à C₆, de préférence CH₃ ; un groupe alcényle en C₂ à C₆ ; ou un groupe alcynyle en C₂ à C₆ ; ou
(ii) un groupe arylalkyle ou hétéroarylalkyle, de préférence phénylalkyle, le groupe alkyle dans ledit groupe arylalkyle ou hétéroarylalkyle étant un groupe alkyle en C₁ à C₄, le groupe phénylalkyle étant de préférence CH₂C₆H₅ ;
R₂ est
(iii) absent dans le cas où Z est O ; ou
(iv) H dans le cas où Z est N ;
ou

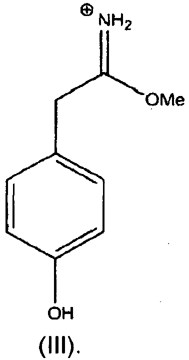

13. Composé(s) pour une utilisation selon la revendication 12, où ledit ou lesdits plusieurs composés sont les seuls agents pharmaceutiquement actifs à utiliser dans ledit traitement ou ladite prévention.

14. Composé(s) pour une utilisation selon la revendication 12 ou 13, où ladite maladie et ledit parasite sont, respectivement :
(a) *Plasmodium* et malaria, ledit parasite étant de préférence *Plasmodium falciparum* ;
(b) *Toxoplasma gondii* et toxoplasmose ;
(c) *Eimeria* et coccidiose ;
(d) *Isospora* et isosporose/coccidiose ;
(e) *Babesia* et babésiose ;
(f) *Cyclospora* et cyclosporose ;
(g) *Cryptosporidium* et cryptosporidiose ;
(h) *Theileria* et theilériose ;
(i) *Neospora* et néosporose ;
(j) *Sarcocystis*/*Hoareosporidium* et sarcocystose.

15. Composé(s) pour une utilisation selon l'une quelconque des revendications 12 à 14, dans lequel ou lesquels A est H.

16. Composé(s) pour une utilisation selon l'une quelconque des revendications 12 à 15, dans lequel ou lesquels B est H.

17. Composé(s) pour une utilisation selon l'une quelconque des revendications 12 à 16, dans lequel ou lesquels ZR₁R₂ est un groupe isoquinolin-1-yle, ledit groupe isoquinolin-1-yle étant au moins partiellement hydraté dans le cycle contenant N, de préférence totalement hydraté dans le cycle contenant N.

18. Composé(s) pour une utilisation selon l'une quelconque des revendications 12 à 14, dans lequel ou lesquels Z est N.

19. Composé(s) pour une utilisation selon l'une quelconque des revendications 12 à 14, dans lequel ou lesquels X est NH, A est COOH, B est H, CH₂OH ou CH₂NH₂, de préférence H et ZR₁R₂ est un groupe isoquinolin-1-yle, ledit groupe isoquinolin-1-yle étant au moins partiellement hydraté dans le cycle contenant N, de préférence totalement hydraté dans le cycle contenant N.

20. Composé(s) pour une utilisation selon l'une quelconque des revendications 12 à 14 ou 19, dans lequel ou lesquels A est COOH, ledit COOH étant en position ortho par rapport à Y.

21. Composé(s) pour une utilisation selon l'une quelconque des revendications 12 à 20, dans lequel ou lesquels l'atome de carbone portant le groupe B est de configuration (S).
